(19) **Europäisches Patentamt / European Patent Office / Office européen des brevets**

(11) **EP 3 283 221 B1**

(12) **EUROPEAN PATENT SPECIFICATION**

(45) Date of publication and mention
of the grant of the patent:
**07.09.2022 Bulletin 2022/36**

(21) Application number: **16724707.1**

(22) Date of filing: **15.04.2016**

(51) International Patent Classification (IPC):
*G01N 15/12* (2006.01)    *G01N 33/487* (2006.01)
*G01N 15/10* (2006.01)    *G01N 15/14* (2006.01)
*B01L 3/02* (2006.01)

(52) Cooperative Patent Classification (CPC):
**B01L 3/0275; G01N 15/12; G01N 15/1459;
G01N 33/48721;** B01L 2200/12; B01L 2300/044;
B01L 2300/0645; B01L 2300/0681;
B01L 2300/0851; G01N 2015/1006;
G01N 2015/1488; G01N 2015/1493

(86) International application number:
**PCT/IB2016/052177**

(87) International publication number:
**WO 2016/166729 (20.10.2016 Gazette 2016/42)**

(54) **DEVICES, SYSTEMS AND METHODS FOR DISPENSING AND ANALYSING PARTICLES**

VORRICHTUNGEN, SYSTEME UND VERFAHREN ZUR AUSGABE UND ANALYSE VON PARTIKELN

DISPOSITIFS, SYSTÈMES ET PROCÉDÉS POUR LA DISTRIBUTION ET L'ANALYSE DE PARTICULES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.04.2015 PCT/IB2015/052754**

(43) Date of publication of application:
**21.02.2018 Bulletin 2018/08**

(73) Proprietor: **Ecole Polytechnique Fédérale de
Lausanne (EPFL)
1015 Lausanne (CH)**

(72) Inventors:
• **BONZON, David Vincent**
**1801 Le Mont-Pélerin (CH)**
• **MULLER, Georges Henri**
**1006 Lausanne (CH)**
• **RENAUD, Philippe**
**1028 Préverenges (CH)**
• **BARRANDON, Yann**
**1026 Echandens-Denges (CH)**
• **BUREAU, Jean-Baptiste**
**1024 Ecublens (CH)**
• **BÉGUIN, Steve**
**Springvale VIC 3171 (AU)**

(74) Representative: **Grosfillier, Philippe
ANDRE ROLAND S.A.
IP Attorneys & Services
Avenue Collonges 21
1004 Lausanne (CH)**

(56) References cited:
WO-A1-01/11338          WO-A1-2015/056176
WO-A2-2005/121780       WO-A2-2010/093998
US-A- 3 714 565

## Description

### Technical Field

The present invention relates to devices, systems and methods to dispense, detect and analyse at the same time fluids and particles contained therein.

### Background Art

Pipette tips and pipettors are extensively used in laboratory practice to precisely handle fluids and particles comprised therein. Pipettors are tools commonly used in chemistry, biology and medicine to transport a measured volumes of liquid, often as a media dispenser, and are usually made of a fluidic pump actuated by a plunger controlling the volume of handled fluid. The tip, also known as pipette tip, is a usually single-use plastic-made tool adapted to work with a pipettor in order to come into contact with a fluid and retaining it. Although standard pipettor systems and pipette tips are very precise in controlling the volume of handled liquid, they do not provide information or control about elements contained in the fluid, such as particles or cells.

Means to detect particles in a fluid were first described in the Coulter counter principle (US2656508A). This principle allows for characterizing dielectric particles in a fluid in term of number and size. The basic components necessary to build a Coulter counter are: i) two chambers filled with conductive saline medium, ii) an orifice separating the two chambers and iii) one electrode immersed in each chamber. When an electrical current is applied between the two electrodes, most of the electrical resistance or impedance is in the orifice. If a cell passes through the orifice, it displaces an equivalent volume of saline resulting in an increase in impedance. However, this prior art does not describe an embodiment suited for integration on a pipette tip.

In a second document (US3714565A), Coulter described an aperture tube in the form of a nozzle for use with a Coulter type particle analysing device having its interior surface covered with conductive material such as metal, its exterior surface covered with conductive material such as metal, the aperture being provided in a corundum wafer set into the bottom end of the tube. The conductive coating approaches and come very close to the aperture, surrounding it except that for the path of the aperture itself, and can act as electrodes for impedance analyses. Such a non-universal design obliges to manufacture a tube having a nozzle-like shaped distal end, with the additional constraint of placing the electrodes closed to the aperture.

Later the concept of coupling of a Coulter counter to a pipettor was proposed by Gascoyne et al. (WO2005/121780 A2). It describes a pipettor system comprising a tip having an orifice, a plunger, a motor, a controller, a display, a first host system, a second host system, a battery, sensor electronics and input mechanism. The orifice may include one or more sensors at or in proximity to the orifice such as one or more impedance sensors that act as fluid passes through, into, or out of it. The orifice may be sized according to knowledge and practice in the art. However, no details are given concerning the design of the tip, the size of the orifice, different sensor settings or the possibility to scale down the analysis up to single cells.

As no details are provided in the document on the specific cell sensor, it is not possible to assess whether the disclosed system is suitable for samples containing small amounts of cells. For this specific application, the design has to be carefully tailored to avoid cell to get trapped in the structure.

WO 2010/093998 A2 discloses a pipette tip adapted to be loaded with a conductive medium comprising particles and to dispense said particles-containing conductive medium, the pipette tip comprising a) a proximal end; b) an elongated body adapted to retain a particle-containing conductive medium; and c) a distal end having a flow opening adapted to dispense a particle-containing conductive medium, said distal end being closed at its extremity by a membrane having an orifice thereon, said orifice being shaped to permit the passage of the medium and the particles one at a time.

Despite the huge amount of work and the advancements in the field at stake, there is still the need of a simple tool specifically answering the problem of single cell dispensing and isolation for procedures in which the isolated cell(s) is (are) going to be further manipulated, cultivated and/or analysed.

### Summary of invention

The present invention overcomes the drawbacks of the prior art by providing devices, systems and methods for dispensing particles such as cells and analysing at the same time one or more characteristics thereof, such as number, size, viability, dielectric properties and the like, through an impedance-based sensor, thus exploiting the Coulter counter principle. A system according to the present invention comprises electrodes, a controller operably connected with a fluidic actuator and an impedance analyser, these latter both operably connected to an aperture tube having a single orifice on an end-tip membrane, named in the frame of the invention "restricted tip".

The aperture tube is typically a fluid retaining tip, hereinafter referred to as a "pipette tip". The system further comprises an electrical impedance analyser comprising at least two electrodes located respectively outside and inside said pipette tip, and operably connected with the controller. When in function, the pipette tip is loaded with a conductive medium, comprising particles to be analysed, in which one of the electrodes is at least partially immersed. The electrodes are used to establish a determined electric field so that a current can flows between the inner and the outer electrodes. Once the loaded pipette tip is immersed in a reservoir which comprises a conductive medium and the second electrode at least partially immersed therein, both current and particles are forced to flow through the orifice, thus flowing from the inside of the pipette tip into the reservoir. The sensing area of the pipette tip is precisely located within the tip thereof, at the frontier with the external conductive medium, which results in the absence of any dead volume. Knowing the electrical field and measuring the current, each single particle flowing outside (or inside) of the tube's sensing area can be detected and analysed via e.g. impedance spectroscopy or coulter counting. For this purpose, a particle detector, such as a time-resolved impedance analyser, is used.

One of the most important features of the invention resides in the particular design of the pipette tip, which is adapted in order to optimize the particles' dispensing and analysis up to single cell level, while avoiding the need of placing the sensor electrodes in precise positions, such as for instance at or in proximity to the orifice. In fact, in order to maximize the change in impedance signal-to-noise ratio due to particle passage, an important parameter to be considered is the dimension of the orifice, which must be small enough to funnelling the electric current and thus concentrate its density within the sensing area and permitting the passage of single particles; however, if such an orifice is located on a much bigger surface area, the distance between the electrodes and the sensing area defined by a much bigger section, and therefore the position of the electrodes, will become less important for the signal-to noise ratio, since a much smaller serial resistance in the particles' containing conductive medium will be present: minimizing this serial resistance will increase the particle sensitivity by improving the signal-to-noise ratio measurement as it consists of measuring variation of the change of the aperture impedance in series with the serial resistance.

Such a design has been facilitated, in parallel, thanks to a novel manufacturing concept applied to the aperture tube, which exploits a process for creating a thin dielectric membrane on the distal end of the pipette tip (which borders the sensing area) and creating later on a small orifice on its surface. Thus, the manufacturing process has been expressly construed in order to positively influence both the design of the aperture tube and the liberty concerning the sensor electrodes' placement. Moreover, this manufacturing process is cheaper and easier compared to those known in the art, and it is readily applicable and adjustable to most of the aperture tube actually in the trade, particularly to disposable plastic pipette tips usually employed in cell handling, thus rendering such a pipette tip design easily accessible to cell biology researchers.

Accordingly, it is an object of the present invention to provide for a pipette tip comprising :

   a) a proximal end possibly equipped with a sterility filter;
   b) an elongated body adapted to retain a particle-containing conductive medium; and
   c) a distal end having a flow opening being closed at its extremity by a membrane having a single orifice thereon adapted to dispense a particle-containing conductive medium and particles comprised therein, said orifice having a diameter comprised between 1 $\mu$m and 1000 $\mu$m; characterized in that the membrane diameter/orifice diameter ratio is of at least 6.32, preferably at least 10.

In a preferred embodiment, the pipette tip has a surface area of the proximal end that is bigger than the surface area of the distal end.

In one embodiment, the pipette tip has a proximal end surface area/distal end surface area ratio of at least 5.

In one embodiment, the membrane of the pipette tip has a thickness comprised between 1 and 1000$\mu$m.

In one embodiment, the pipette tip has a membrane's diameter/membrane's thickness ratio of at least 5.

In one embodiment, the orifice of the membrane's pipette tip is a slotted hole, a funnel or a diaphragm.

In a preferred embodiment, the pipette tip has a proximal end adapted to be operably connectable with both a fluidic actuator and an electrical impedance analyser.

In one embodiment, the pipette tip comprises at least one electrode physically connected to a portion of the elongated body and/or of the distal end and/or the membrane and/or a filter, said electrode being electrically activated once the pipette tip is operably connected to an electrical impedance analyser.

In one embodiment, the pipette tip comprises at least two electrodes adapted to be operably located respectively outside and inside it.

In a preferred embodiment, the pipette tip has a substantially frustum shape.

In a preferred embodiment, the pipette tip is made of a biocompatible material.

In a preferred embodiment, the pipette tip is sterilisable.

In a preferred embodiment, the pipette tip is made of a plastic material.

In a preferred embodiment, the pipette tip is disposable.

In a preferred embodiment, the membrane of the pipette tip is substantially composed of a polymeric plastic material.

In another aspect of the invention, it is provided a process for manufacturing a plastic pipette tip comprising a proximal end, an elongated body adapted to retain a particle-containing conductive medium and a distal end having a flow opening being closed at its extremity by a membrane having a single orifice thereon having a diameter comprised between 1 $\mu$m and 1000 $\mu$m, wherein the membrane diameter/orifice diameter ratio is of at least 6.32, said process comprising the steps of:

> a) injecting a liquid plastic material into a mold defining the elongated body's walls, form and thickness so to obtain a plastic pipette tip;
> b) providing a closed membrane;
> c) opening a single orifice on the membrane;
> d) sealing the membrane to the distal end of the plastic pipette tip; and
> e) optionally applying at least one electrode on the plastic pipette tip so that this latter can be operably connectable with an electrical impedance analyzer

wherein steps a) and steps b)-c) together are interchangeable.

In a further aspect of the invention, it is provided a process for manufacturing a plastic pipette tip comprising a proximal end, an elongated body adapted to retain a particle-containing conductive medium and a distal end having a flow opening being closed at its extremity by a membrane having a single orifice thereon having a diameter comprised between 1 $\mu$m and 1000 $\mu$m, wherein the membrane diameter/orifice diameter ratio is of at least 6.32, said process comprising the steps of:

> a) injecting a liquid plastic material into a mold defining the elongated body's walls, form and thickness so to obtain a plastic pipette tip;
> b) temporary closing the distal end of the so-obtained plastic pipette tip;
> c) depositing a film of a plastic material on the elongated body's walls and the distal end of the plastic pipette tip, thus defining a membrane at the extremity of said distal end;
> d) removing the closure at the distal end of the plastic pipette tip;
> e) opening a single orifice on the membrane; and
> f) optionally applying at least one electrode on the plastic pipette tip so that this latter can be operably connectable with an electrical impedance analyzer.

A still further aspect of the present invention relates to a plastic pipette tip manufactured through the above-described methods, and having all combinations of features of the pipette tip previously described.

A still further aspect of the present invention relates to a system for use in dispensing and analysing particles through impedance-based means, characterized in that it comprises:

> a) a pipette tip as described above;
> b) an electrical impedance analyser adapted to be operably connectable to said pipette tip;
> c) a fluidic actuator adapted to be operably connectable to the proximal end of the pipette tip; and
> d) optionally a controller adapted to be operably connectable to, and operate, both the fluidic actuator and the electrical impedance analyser.

In one embodiment, the system further comprises a computer-like device operably connected to the electrical impedance analyser for analysing and/or store impedance data.

In a preferred embodiment, the electrical impedance analyser comprises electrical integrated circuits according to the lock-in demodulator principle.

In one embodiment, the fluidic actuator comprises at least one pressure source and one regulator for modifying the pressure inside the pipette tip.

In a one embodiment, the fluidic actuator comprises two pressure sources and one regulator for modifying the pressure inside the pipette tip.

In one embodiment, the fluidic actuator comprises at least one air pump for modifying the pressure inside the pipette tip.

In a preferred embodiment, at least one air pump is a fast switch pump.

In one embodiment, the fluidic actuator comprises at least two air pumps operably connected to the pipette tip through a 3-ways valve adapted so that at least one pump generates a positive pressure and at least one pump generates a negative pressure inside the pipette tip.

In a preferred embodiment, the pump is microfabricated piezo actuated membrane pump. The small dead volume compared with the working volume combined with the fast membrane actuation of the piezo allows a fast switching between two different pressures as required for the application.

In one embodiment, the system comprises a hydrostatic pressure sensor placed within the pipette tip and in proximity of the orifice.

In one embodiment, the controller is programmed with an algorithm so that it regulates the fluidic actuator activity in response to the measurements performed by the electrical impedance analyser.

**Brief description of drawings**

Figure 1 shows a cross-section of the restricted tip;
Figure 2 shows the restricted tip connected to the system for particle dispensing and analysis;
Figure 3 shows the sensing tip and its multiple electrodes arrangements;
Figure 4 shows the sensing tip connected to the system for particle dispensing and analysis;
Figure 5 shows the connector and illustrates how it connects with the sensing tip;
Figure 6 illustrates the sealing process of the membrane on a conventional tip;
Figure 7 illustrates the layer deposition process using a temporary external closing element;
Figure 8 illustrates the layer deposition process using a temporary internal closing element;
Figure 9 shows the block diagram of one embodiment of the fluidic actuator;
Figure 10 shows the block diagram of another embodiment of the fluidic actuator;
Figure 11 shows the block diagram of the impedance analyser;
Figure 12 shows the algorithm for particle dispensing and analysis;
Figure 13 illustrates the sequence of events for dispensing and analysis of particles;
Figure 14 shows a graph based on a simulation for a micrometric bead flowing through a cell sensor according to the invention in the form of a nozzle-like aperture or of a holed membrane. The graph highlights the high impedance sensitivity dependence on the electrodes positioning for the nozzle-like topology compared to the holed membrane;
Figure 15 shows one embodiment of a sensing tip according to the invention;
Figure 16 (a) shows a recording graph of the normalized impedance of 6 $\mu$m-beads flowed through a sensing tip. Each peak corresponds to the passage of a particle that can be clearly distinguished; (b) Magnified impedance peak of one single bead.
Figure 17 shows an impedance graph of the sensing tip according to the invention for different electrodes position inside the tip itself.

**Description of embodiments**

The present disclosure may be more readily understood by reference to the following detailed description presented in connection with the accompanying drawing figures, which form a part of this disclosure.

As used herein and in the appended claims, the singular forms "a," "and," and "the" include plural referents unless the context clearly dictates otherwise. Thus, for example, reference to "a cell" includes a plurality of such cells and reference to "an electrode" includes reference to one or more electrodes, and so forth.

Also, the use of "or" means "and/or" unless stated otherwise. Similarly, "comprise," "comprises," "comprising" "include," "includes," and "including" are interchangeable and not intended to be limiting. It is to be further understood that where descriptions of various embodiments use the term "comprising", those skilled in the art would understand that in some specific instances, an embodiment can be alternatively described using language "consisting essentially of" or "consisting of."

**TIP STUCTURE**

As used herein, a "pipette tip" refers to a tool (named tip) usually used in combination with a pipettor or chemical dropper, a laboratory tool used in chemistry, biology and medicine to transport a measured volume of liquid, often as a media dispenser. A tip has an elongated, substantially tubular body that has a bottom opening (hereinafter, "distal end") at the bottom end for the flow passage of a liquid, a top opening (hereinafter, "proximal end") at the top end for the passage of air, and a passageway (hereinafter, "elongated body") between the bottom opening and top opening for the retention of a liquid inside the tip defined by at least one delimiting wall. A pipette tip is characterized by a tubular body usually consisting of a transparent or translucent material such as glass or a (thermo)plastic material which is of a substantially cylindrical, conical or frustum (such as frusto-conical or frusto-pyramidal) shape, even if other topologies can be envisaged such as a nozzle-like design. A pipette tip can comprise at its proximal end a filter which ameliorates the sterility thereof once coupled with a fluidic actuator such a pipettor, but in the frame of the present invention it can also act as a support for placing at least one electrode as described below in more details.

A pipette tip, such as those of the invention, can be molded as a unitary, integral body of plastic from any suitable standard polymer material known in the art (e.g., polypropylene, polystyrene, polycarbonates, polysulfones, polyesters, cyclic olefins and so forth) using well-known injection molding methods. However, other materials, as well as further manufacturing methods, can be envisaged for the manufacturing of the article, such as for instance glass. The polymeric material chosen to form the tip must be nonreactive with, insoluble in, and impervious to the materials that come into contact with the pipette tip, such as cells, media such as culture media and/or further chemicals/biologic materials. Actually, a pipette tip can be made of any suitable material as long as it keep the ability of retaining and release its content on demand. Preferably, a pipette tip is a plastic pipette tip which is biocompatible, preferably also easily sterilisable (such as e.g. with irradiation by gamma-rays) and preferably also disposable, in order to avoid (cross)contamination with several samples and/or environmental pollutants.

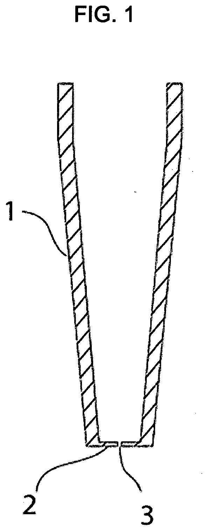

A tip according to the present invention (Fig. 1) comprises a body 1 and a thin membrane 2 which closes the extremity of the distal end. In the frame of the present disclosure, a "membrane" is a blocking element which is placed at the opening of the distal end of a pipette tip and completely impedes the flow of a liquid through it. More precisely, in the present document, the membrane has to be understood as a substantially planar element, such as a disc, located within the tip distal end. The membrane surface is therefore approximatively equivalent to the internal surface of the tip distal opening. Such a membrane may be therefore defined as a floating element because its surface is not fixed to the tip body 1. The membrane is characterized by a specific ratio between its diameter and its thickness, which must be of at least 5, preferably at least 10 and most preferably at least 20. Said membrane has on its surface a small orifice 3 which is shaped in order to permit the passage, during a dispensing phase, of a liquid such as an electrical conductive medium and of particles (such as for instance cells or micro/nanobeads) comprised therein one at a time, provided that the size and the shape of the orifice 3 is such to permit the passage of said particles. Such a tip will be referred to hereinafter also as a "restricted tip".

For the sake of clarity, a "dispensing phase" as used herein refers to a process of releasing a conductive medium contained within the tip of the invention through the application, as will be detailed later on, of a positive pressure inside the tip, allowing the flow and therefore the release of at least a part of its content. However, for "dispensing phase" is also herein meant, *mutatis mutandis,* the opposite process of aspiring a conductive medium, possibly comprising particles dispersed therein, from a container or a reservoir into the body 1 of the tip, through application of a negative pressure within the tip itself. A skilled person would easily envisage how to adapt the system and the methods according to the invention in order to obtain the desired dispensing phase, either in releasing or aspiring mode. The use of the wording "dispensing", "dispensing phase" and the like is exclusively used for conciseness and ease of description, but should be intended to refer to both meaning.

As used herein, an "orifice" is any opening, hole or vent that completely crosses a physical body so that the volumes at the two sides of said body are brought into communication among them. Particularly, an orifice 3 according to the present disclosure completely crosses the membrane 2 at the tip's distal end by creating a channel that is substantially cylindrical or conical in shape so that the internal volume of the pipette tip and the outside are fluidically connected. In its simplest embodiment, an orifice is a cylinder sized and shaped so that a liquid and a particle, such as a cell, comprised therein can passed through it. However, several variants of such an orifice can be envisaged, some of which are particularly useful in order to avoid the clogging thereof and/or to avoid the reentry of a particle once it has been dispensed, such as a slotted hole, a funnel or a diaphragm-like shape. The orifice of the membrane's pipette tip has a diameter comprised between 1 and 1000$\mu$m, which is a suitable size for most of the particles (such as cells) usually analyzed via impedance-based means.

The addition of a holed membrane at the extremity of a pipette tip represents the core inventive concept behind the present invention. In fact, a such-designed tip is particularly suitable and optimized to perform highly reliable and accurate measurements, preferably impedance measurements, once said tip is coupled with a system for dispensing, and analysing the characteristics of (e.g. an impedance analyser), the particles comprised in a conductive medium, especially up to a single-particle level. This approach has huge potential advantages and applications for examples in single cell research or protein production for medicament manufacturing, where selecting a single cell, possibly with precise features, is crucial for a good outcome.

The membrane 2 is a key element in the structure and design of the pipette tip of the invention. The structure of the membrane 2 and of the orifice 3 thereon simplifies the positioning of the electrodes (one inside and one outside the tip, both at least partially immerged in conductive media) when the tip is intended to be coupled with an impedance-based system for analysing particles flowing throughout it. In fact, for its intrinsic characteristics, such a design permit not to place the electrodes in a precise and particular arrangement, such as on the body tip and/or the membrane, and in any case not very closed to the orifice 3. Moreover, the membrane 2 defines the frontier of the sensing area, giving no incertitude between what is detected and what is dispensed in e.g. a medium-filled reservoir, allowing high confidence measurements particularly for single cell dispensing and analysis.

Impedance analyses, and particularly the sensibility thereof, are influenced by the electrical current density at the orifice's 3 level. In fact, the orifice 3 represents the frontier between the conductive medium placed inside the tip and a medium in an external reservoir fluidically connected with the pipette tip, thus creating a passage through which the electrical current is forced to pass in order to close the electrical circuit established by the activated electrodes. In such a way, a particle traversing the orifice 3 will "interrupt" or in any case alter the electrical circuit, thus providing a detectable signal. In a first approximation, the detectable signal is proportional to the variation of resistance in the orifice divided by the sum of the resistances in series in the electrical circuit including the resistance of the conductive medium in and out of the sensing tip. The sensitivity can be approximated by the following formula:

$$sensitivity = \frac{\Delta R_{orifice}}{R_{orifice} + R_{inside} + R_{outside}}$$

Consequently, the sensitivity is improved when the resistance inside and outside the restricted tip are low compared to the resistance at the orifice. The resistance of a resistor depends upon its length and cross sectional area. This is regulated by the resistivity law:

$$R = \rho \frac{l}{a}$$

where "l" is the length of the conductor (in this case, the liquid height inside the tip), "a" is the cross-sectional area (of the membrane; for a round conductor a = $\pi r^2$ if r is radius) in units of meters squared, and "p" is the resistivity of the conductor (in this case, the conductive medium). According to this formula, the resistance is thus mostly influenced by the cross-sectional area of the distal end.

In order to achieve a good sensitivity, it is then best to design the pipette tip with a large cross-sectional area at the distal end, a large membrane 2 and a small orifice 3 with a diameter in the magnitude of the particle size. In addition, choosing such a design gives some freedom on the placement of the electrodes, because the length "l" has a linear impact in the equation and is thus much less influent than the cross section, which has a quadratic influence. Therefore, the greater the membrane area will be, the least the electrode placement will be important.

In the attempt of improving the pipette tip design for impedance particle analyses, the inventors have been able to define that the optimal membrane diameter/orifice diameter ratio should be of at least 6.32, preferably of at least 10. Coulter et al. proposed a design of a Coulter counter at the distal part of a nozzle (US3714565A). This solution is known to allow cell detection with characteristic length between inner electrode and an aperture in the range of the cell size. However, to obtain such a topology, an expensive microfabrication process including photolithography is necessary. Avoiding photolithography, imposes an increase in the fabrication tolerance. In this context, the electrode position can change in a range spanning from 50 to 500μm (one order of magnitude); therefore, in order to have similar performance than that proposed by the Coulter counter nozzle, the electrical resistance inside the tip above the membrane should remain unchanged.

Assuming that the length L between the suspended (floating) membrane and an inner electrode is at least 10 time less controllable in a manufacturing process than in the Coulter's nozzle design:

$$L_{suspended\ membrane} = 10 * L_{nozzle}$$

There is the need to keep the same electrical resistance inside the tip for the cell sensor of the present invention to work

$$R_{inside} = \frac{\rho L_{nozzle}}{S_{nozzle}} = \frac{\rho L_{suspended\ membrane}}{S_{suspended\ membrane}} = \frac{\rho * 10 * L_{nozzle}}{S_{suspended\ membrane}}$$

The surface of the suspended membrane is therefore:

$$=> S_{suspended\ membrane} = \frac{\rho*10*L_{nozzle}}{R_{inside}} = \frac{\rho*10*L_{nozzle}}{\frac{\rho L_{nozzle}}{S_{nozzle}}} = 10*S_{nozzle}$$

And its radius is therefore :

$$=> r_{suspended\ membrane} = \sqrt{\frac{S_{suspended\ membrane}}{\pi}} = \sqrt{\frac{10*S_{nozzle}}{\pi}} = \sqrt{\frac{10*r_{nozzle}{}^2*\pi}{\pi}} =$$

$$\sqrt{10*r_{nozzle}{}^2} = \sqrt{10}*r_{nozzle} = 3.16*r_{nozzle}$$

As described in the above-mentioned US3714565A, the Coulter design gives

$$r_{nozzle}=2*r_{aperture}$$

$$r_{suspended\ membrane} = 2*3.16*r_{nozzle} = 6.32*r_{aperture}$$

And therefore:

$$d_{suspended\ membrane} = 2*3.16*d_{nozzle} = 6.32*d_{aperture}$$

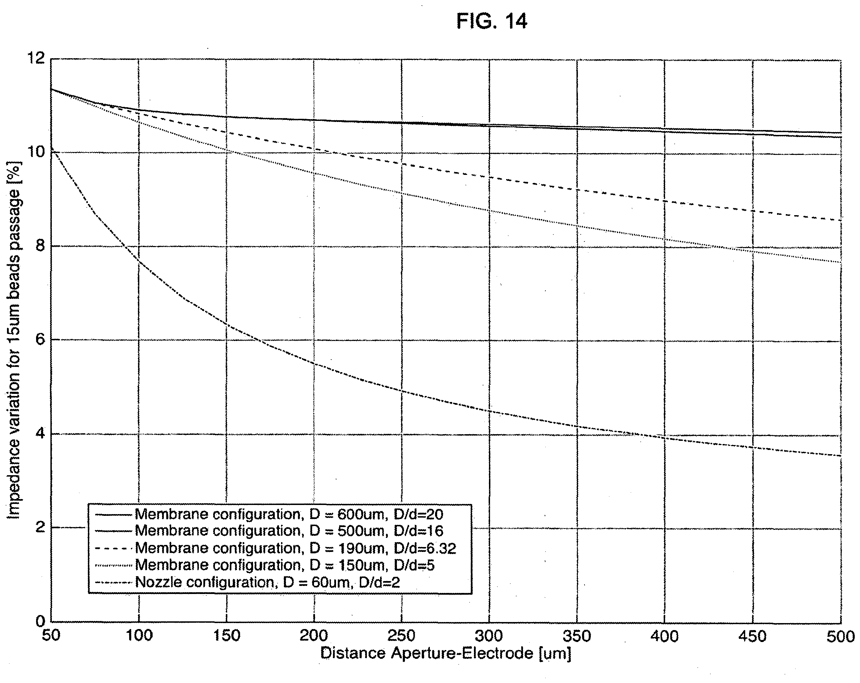

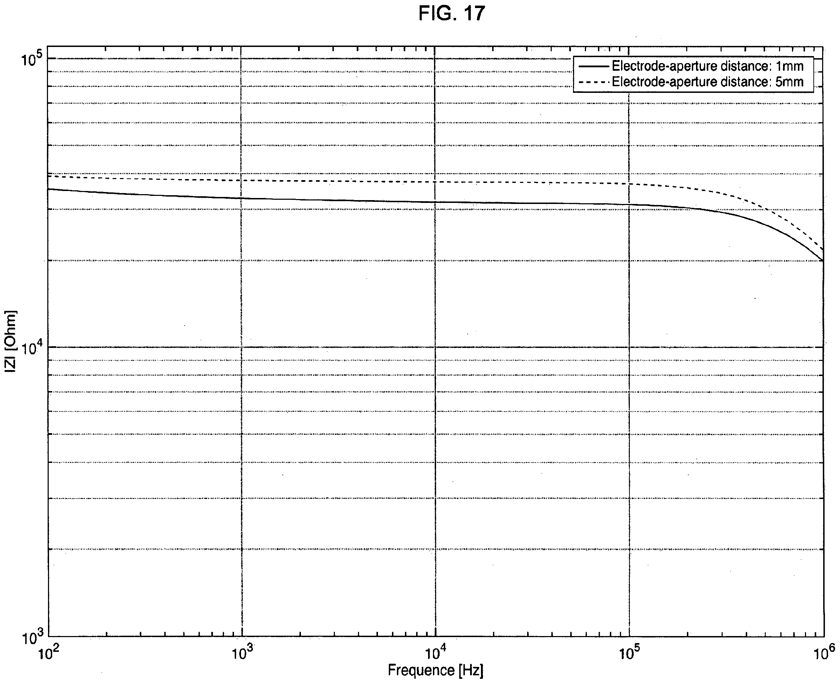

This result justifies the choice of a precise ratio between the membrane diameter and the orifice size, which must be of at least 6.32, for having enough freedom in electrode positioning while keeping an excellent performance in terms of sensitivity, and without being linked to micromanufacturing processes. However, as shown in Figure 14, a surprising effect is particularly given when said ratio is of at least 15, even better with a ratio of 20 (Figure 14). This effect is further experimentally demonstrated in Figure 17; here, the plain curve shows the case of an inner electrode placed at 1mm from the aperture, and the dotted curve the case of electrode placed at 5mm from the aperture. The difference of impedance in the resistive plateau if less than 20% (37kOhm for the 5mm distance and 31kOhm for the 1mm distance with measurement at 50kHz). The figure clearly demonstrates that even a large electrode displacement in the tip does not change the order of magnitude of the tip impedance.

A continuous calculation based on the above mentioned considerations and on a theoretical model developed by the inventors was obtained in the case of an orifice placed in a membrane and of an aperture at the end of a nozzle. Fig 14 shows the simulation results of impedance variation due to the passage of 15 $\mu$m polymeric beads through an impedance sensor in function of the distance between the aperture and an electrode placed inside an aperture tube for two different embodiments. In the depicted image, a 30 $\mu$m (d) hole has been included in a 500 $\mu$m (D) membrane (D/d=16,6) and a 30 $\mu$m (d) hole has been included at the end of a 60 $\mu$m (D) nozzle (D/d=1,66). This figure highlights that the effect of the electrode placement in terms of particle sensitivity becomes significant in the case of small D/d ratio. The proposed design with larger D/d ratio has the advantage of more flexibility for electrode placement with limited effect on particle sensing sensitivity.

The membrane 2 can be made of any suitable material, which is preferably a (thermo)plastic polymeric material commonly used for pipette tips such as polypropylene, polystyrene, polycarbonate, and the like. Preferably, the membrane 2 of the pipette tip has a thickness comprised between 1 and 1000$\mu$m and a membrane's diameter/membrane's thickness ratio of at least 5. These parameters have been established by the inventors as the optimal ones in order to obtain an excellent impedance readout while maintaining a good resistance of the membrane 2 to hydrostatic and/or applied pressures.

At the same time, the surface of the proximal end of the pipette tip should be bigger than the surface of the distal end, preferably with a ratio of a least 5. For this reason, in a particular embodiment of the invention (such as the one shown in Fig. 1), the pipette tip of the invention has a substantially frustum three-dimensional volume shape. As used herein, a "frustum" is a portion of a solid, such as a cone or a pyramid, which lies between two parallel planes cutting it. Each plane section is a floor or base of the frustum. A frustum is circular if it has circular bases; it is right if the axis is perpendicular to both bases, and oblique otherwise. The height of a frustum is the perpendicular distance between the planes of the two bases. For example, in one embodiment, the pipette tip comprises a single rounded wall connecting the proximal and the distal ends, and a bottom distal end surface represented by the holed membrane 2, the height of the pipette tip being the distance between the proximal end opening and the bottom membrane surface area. Accordingly, the tip is substantially frusto-conical in shape. In an alternative embodiment, the pipette tip comprises three or more wall faces, and the volumetric shape may therefore be substantially frusto-pyramidal.

The restricted tip of the invention has been particularly conceived and adapted in order to best perform in the frame of impedance analyses once coupled with a system comprising at least an impedance analyser and a fluidic actuator, as discussed below in more details.

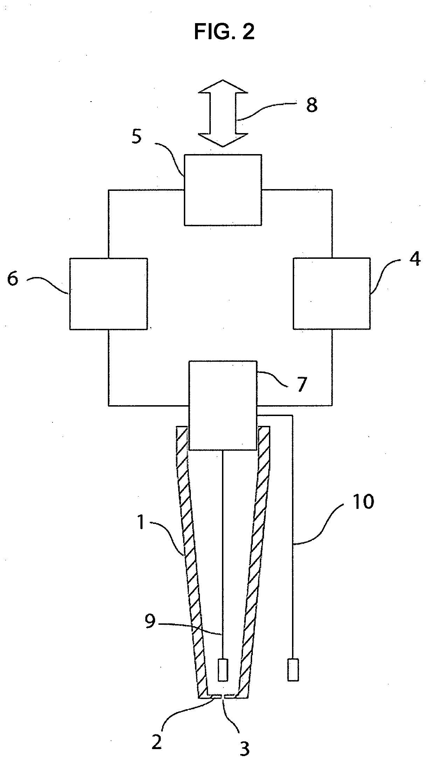

For example, as shown in Fig. 2, a restricted tip can be operably connected to a system comprising a fluidic actuator 6 and an impedance analyser 4 via e.g. a tip connector 7 having at least two electrodes by locking the tip so that one of the electrodes (9) falls inside the body of the tip while the other (10) lies outside the tip. In such an arrangement, the two electrodes 9, 10 are so called "floating" or "connecting" electrodes, adapted to be at least partially immerged into the conductive media comprised in the tip and in a reservoir fluidically connected with the tip's inside.

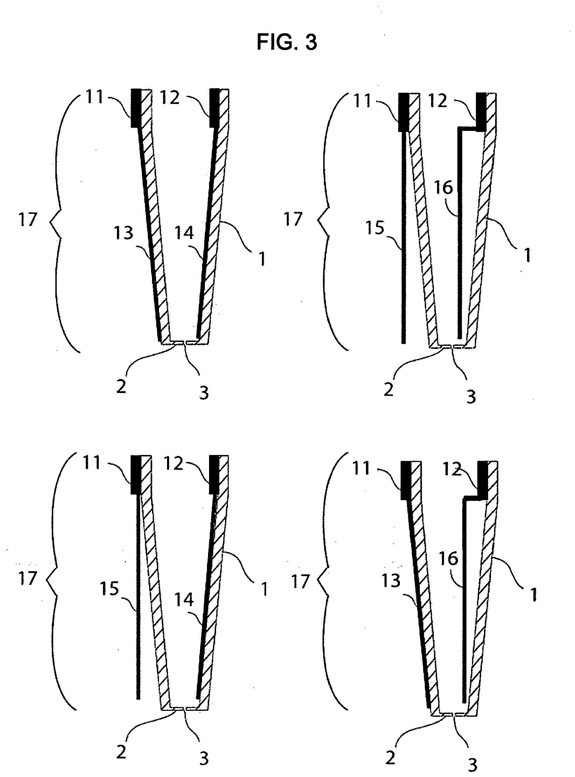

Alternatively, the restricted tip can be implemented by physically connecting or attaching at least one electrode to a portion of the elongated body, the distal end, the tip membrane and/or a filter in a manner to activate said at least one electrode once the tip is operably connected to the electrical impedance analyser 4 through a tip connector 7. Such an implemented restricted tip is referred to hereinafter as a "sensing tip" 17 (Fig. 3).

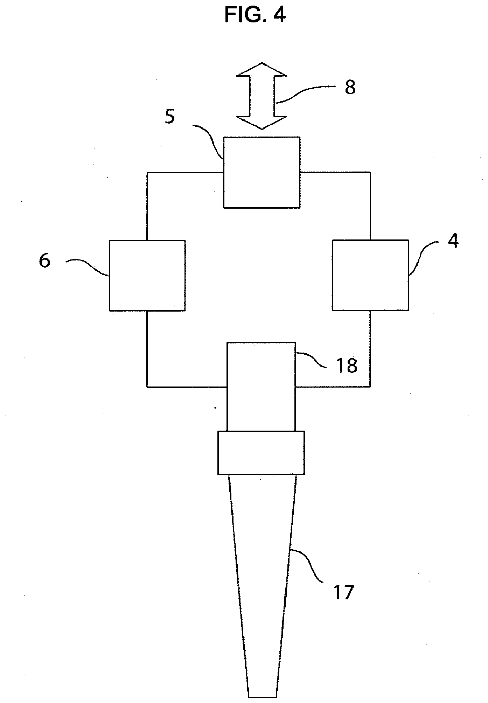

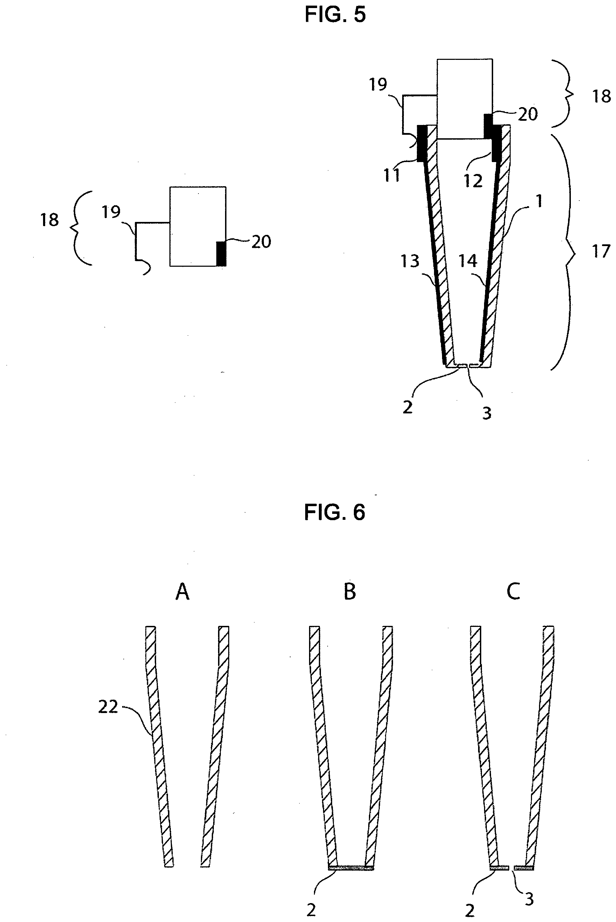

Also in this case, in view of the inventive design of the pipette tip of the invention, many alternatives concerning the placement of the electrodes can be envisaged, thus providing not only a great operational freedom so that many diverse impedance analyser can be used for particle analyses, but also the possibility to adapt the manufacture process in order to be the more comfortable as possible, depending on the needs and the tools at one's disposal. As depicted in Fig. 3 for instance, several different arrangements of the electrodes can be imagined, wherein the electrodes lie along the internal or external walls of the tip's body **(13, 14),** remain floating **(15, 16)** or combinations thereof. The electrodes can also be physically connected to the tip's walls via an external **(11)** or external **(12)** connection pad that may facilitate the coupling with the impedance analyser **4** through a tip connector **18** comprising connector pads **(19, 20)** (Figs. 4 and 5). Additionally or alternatively, floating electrodes can be locked within a filter provided at the proximal end of the sensing tip **17**. Additionally or alternatively, the internal electrode can be coupled with or embedded within a further tip element such as a plunger or a capillary piston included inside the tip itself, so that the sensing tip can work as a positive displacement pipette tip such as the ones described e.g. in EP0494735.

The electrodes can be shaped to have any form and can be made of any suitable electrical conductive material, including but not limited to metals such as Au, Pt, Al, Cu and the like, liquid metals such as Hg or Ga, as well as any alloy or oxide thereof, conductive powders or adhesives as well as any combination of the foregoing. In a preferred embodiment, the electrodes are made of non-toxic and biocompatible materials.

For the sake of clarity, in the frame of the present disclosure, the expression "operably connected" reflects a functional relationship between the several components of the system or the sensing tip among them, that is, the term means that the components are correlated in a way to perform a designated function. The "designated function" can change depending on the different components involved in the connection; for instance, the designated function of an electrical impedance analyser 4 operably connected to a restricted tip or a sensing tip is the detection of an impedance signal once a particle flows together with a conductive medium through the orifice **3**. Similarly, the designated function of a fluidic actuator 6 operably connected to a restricted tip or a sensing tip is the regulation of the pressure inside said tip. A person skilled in the art would easily understand and figure out what are the designated functions of each and every component of the system of the invention, as well as their correlations, on the basis of the present disclosure.

**TIP FABRICATION PROCESS**

According to another aspect of the invention, it is provided a manufacturing method for the production of the restricted or the sensing tip described above.

The restricted tip has a very simple structure that is easy to manufacture at large scale while maintaining minimal costs. The machines used for these processes can host batches of hundreds of tips, and in mass production the process can be automated with digital processing software. Generally speaking, in preferred embodiments of the invention, a variety of polymeric plastic materials may be used for manufacturing the pipette tips body **1** and/or membrane **2,** including but not limited to standard thermoplastic polymers such as polypropylene, polystyrene, polycarbonate, and the like.

In one embodiment, a one-step manufacturing process foresees the creation of a pipette tip having its distal end closed with the membrane **2** having an orifice **3** in the form of a monolithic piece of a polymeric plastic material. In this case, the monolithic tip may be manufactured e.g. by plastic injection moulding techniques using ad hoc molds.

In another, two-steps monolithic manufacturing process embodiment, the pipette tip including the membrane 2 is produced by plastic injection moulding techniques. Subsequently, the orifice is opened through the membrane by any suitable means such as laser ablation, punching, etching, drilling and the like. In a preferred embodiment, the orifice is opened through the membrane by an excimer laser (LightShot, OPTEC).

In a three-steps layer deposition manufacturing process embodiment (Fig. 6), the pipette tip is first produced by plastic injection moulding techniques (A). In a currently developed process, commercially available pipette tips **21** are taken as the basis of the manufacturing of the restricted tip (RatioLab Colorless E, 200uL). In a second step (B), the membrane 2, a film-like plastic material such as polypropylene, polystyrene, polycarbonate, and the like, is sealed to the tip's distal end by e.g. welding or gluing techniques. In a third step (C), the orifice 3 is opened through the membrane 2 by any suitable means (laser ablation, punching, etching, drilling and the like). In a preferred embodiment, the orifice is made by an excimer laser (LightShot, OPTEC).

In another embodiment, an indirect layer deposition manufacturing process is used. The tip is first produced by plastic injection moulding techniques. In a second step, the membrane is a film-like plastic materials such as polypropylene, polystyrene, polycarbonate, and the like and then an orifice is opened through the membrane by suitable means as described above. The membrane including the orifice is later sealed to the tip by suitable means as described above ultrasonic (e.g. welding or gluing techniques). In a currently developed process, the orifice opening is made by an excimer laser.

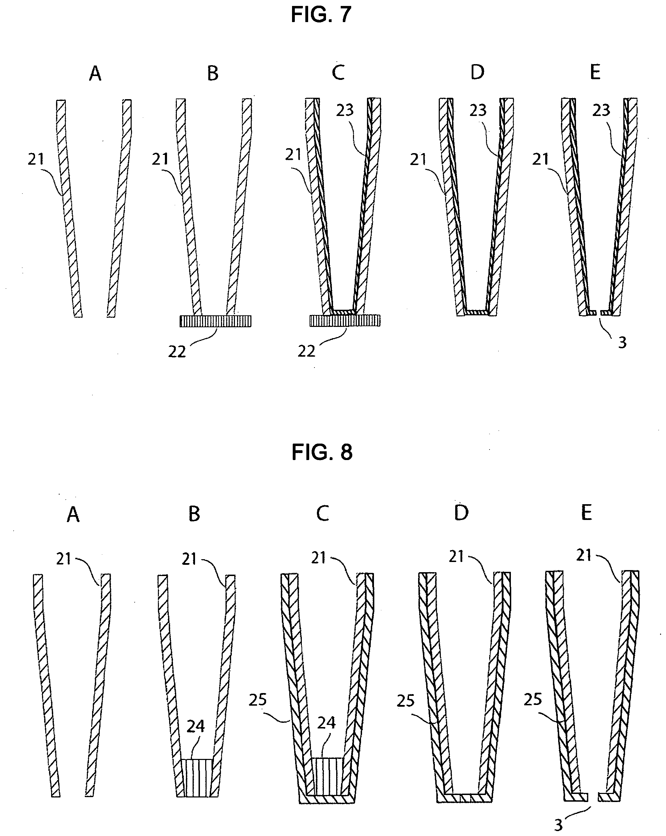

In a five-steps layer deposition manufacturing process embodiment (Fig. 7), the tip 21 is first produced by plastic injection moulding techniques (A) and is later temporary closed at the aperture's level (B) with an external closing element **22** such as a film of e.g. polypropylene, polystyrene, polycarbonate and the like on the external side. In a currently developed process, Parafilm M (Bemis NA) has been chosen because it has an optimal adherence on the tip. Subsequently (C), the membrane **23** is coated on the internal side of the tip and of the temporary external closing element by using any suitable technique such as physical or preferably chemical vapour deposition (CVD) process so that the polymer closes the opening of the tip **21**. In a fourth step (D), the temporary external closing element **22** is mechanically detached. Finally (E), the orifice 3 is opened through the membrane by suitable means as described above.

In an alternative embodiment of the five-steps layer deposition manufacturing process (Fig. 8) the tip **21** is first produced by plastic injection moulding techniques (A). Then (B) the tip **21** is temporary filled with an internal closing element **24** such as a sacrificial plug made of a soluble polymer, for instance poly(ethylene-glycol) which is soluble in water. In a currently developed process, PEG1000 (Sigma Aldrich) is chosen because it is liquid at 50°C and then can be easily loaded inside the tip **21**. It is also easily removable because of its water solubility. In a third step (C), a membrane **25** is coated on the external side of the tip's distal end closed with the internal closing element and possibly also on the external side of the pipette's wall by using any suitable technique such as physical or preferably chemical vapour deposition (CVD) process. The coating can be for instance a conformal polymer such as poly(p-xylylene) (tradename Parylene) deposited with accurate thickness ranging from 50nm to 1mm depending on the application. In a current embodiment, the membrane is made of a biocompatible polymer (Parylene USP class VI). After the coating, the polymer closes the opening of the tip. In a fourth step (D), the internal closing element **24** is removed for example by dissolution in a solvent. Thus, the polymer forms a membrane that closes the pipette tip **21**. In a last step (E), the orifice 3 is opened through the membrane by any suitable method, as those described above.

The same fabrication processes of the restricted tip described above are applicable to the manufacture of the sensing tip according to the invention. In addition to the described steps, (a) last step(s) of positioning and physically applying at least one electrode in the internal and/or external side of the pipette tip is performed (see for instance Fig. 3), together with an optional final step of positioning an air filter. As extensively stated throughout the description, the physical application of electrodes on a restricted tip to obtain a sensing tip can be attained with a great variety of designs and combinations, as well as with various manufacturing techniques, in view of the relative importance of the electrodes' position.

In one embodiment, at least one external electrode is deposited outside of the tip's tubular body 1. The external electrode comprises or consists of e.g. a metal or other suitable conductive material and may be deposited on the surface of the tip by suitable methods such as sputtering or any other deposition method such as metal evaporation. In a currently developed manufacturing method, the external electrode is made of a thin (about 100nm) gold layer that is deposited by evaporation on the external surface of the tip. In mass production, large batch of tips can be placed in the evaporator. The gold layer has been chosen for its excellent adhesion on the tip, particularly plastic pipette tips, and moreover it is well suited for handling biological samples because it is biocompatible. The external electrode can also be for example a simple conductive wire or a conductive adhesive.

In one embodiment, at least one internal electrode is deposited inside the tip's tubular body. Similar considerations as for the external electrode can be applied to the internal electrode, particularly in terms of used materials and deposition methods. In a currently developed manufacturing method, the internal electrode is a simple metal wire placed into the tip and hold in place by an air filter located at the tip's proximal end (floating electrode). Wires made of medical-grade metal may be used, such as stainless steel 316L which is biocompatible and has no electrical insulator outside.

**FLUIDIC ACTUATOR**

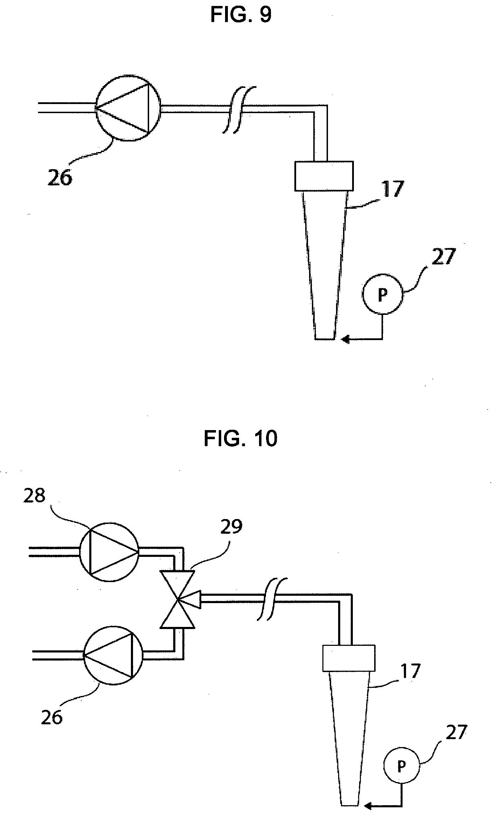

Another important feature of the invention resides in the particular design of the fluidic actuator 6, which is adapted to control the conductive medium and particles' retention or their passage through the tip (Fig. 9).

In its simplest embodiment, the fluidic actuator 6 is any kind of device able to apply a pressure on a tip operably connected thereto. In this embodiment, the fluidic actuator 6 commonly works by exerting, upon activation, a negative pressure change inside a tip connected thereto to aspire a fluid, and selectively releasing said fluid to draw up and dispense it according to a preferred volume by applying a positive pressure change. A syringe or syringe-like device could be suitable for this purpose. In a preferred, alternative embodiment, devices such as a manual or electronic pipettor, as commercially available ones, could be used. Alternatively, the use of a pipettor compatible with positive displacement pipettes can be envisaged.

In another embodiment, the fluidic actuator 6 comprises at least two pressure sources (one positive and one negative) and a regulator capable of applying a controllable negative pressure (also referred to hereinafter as "holding pressure") and a controllable positive pressure (also referred to hereinafter as "dispensing pressure").

In another embodiment (Fig. 9), the fluidic actuator 6 comprises at least one air pump 26 for generating the pressure inside the restricted or sensing tip. In this configuration, the fluidic actuator 6 works by only exploiting the hydrostatic pressure of the internal conductive medium as a dispensing pressure, and the actuator 6 typically applies a negative pressure (also referred to hereinafter as "holding pressure") towards the proximal end of the restricted or sensing tip 17 that sucks a smaller flow compared to the dispensing flow to ensure that no particle can leave the restricted or sensing tip. In this embodiment, the fluidic actuator 6 typically comprises one air pump 26 as for example mircofabricated piezo actuated membrane pump operably connected to the restricted or sensing tip. In order to better perform and rapidly invert the pressure imposed to the conductive medium inside the restricted or sensing tip, the micropump need to be able to establish at least 2mbar (or -2mbar) in the tip's internal volume by adding or removing air from it. This delay (e.g. 100ms) need to be fulfilled in order to obtain a system sufficiently reactive to be able to stop the particles' containing conductive medium dispensing just after the chosen number of particles of interest (e.g. one single cell) has been detected, and without dispensing more than that number.

In another embodiment (Fig. 10), the system comprises at least two air pumps **26, 27** adapted so that at least one pump **26** generates a positive pressure and at least one pump **27** generates a negative pressure inside the restricted or sensing tip **17.** In this embodiment, the fluidic actuator typically comprises two air pumps as for example microfabricated piezo actuated membrane pumps with capability to pump external air and operably connected with the pipette tip **17** of the invention through e.g. a 3 ways valve **28.** In order to better perform and rapidly invert the pressure imposed to the conductive medium inside the pipette tip **17,** the valves **28** need to be able to switch very quickly, preferably in less than 100ms, and the micropump(s) need to be able to establish at least 2mbar (or -2mbar) in the tip's internal volume by adding or removing air from it. This delay (e.g. 100ms) need to be fulfilled in order to obtain a system sufficiently reactive to be able to stop the particles' containing conductive medium dispensing just after the chosen number of particles of interest (e.g. one single cell) has been detected, and without dispensing more than that number.

Moreover, the fluidic actuator 6 needs to work by pumping external air as such a system does not tolerate a liquid contact with the particle media and the pump. For instance, one pump is arranged in a way to pump air from atmosphere into the tip (increasing, positive pressure) and the other pump is arranged so to extract air from the tip's inside into the atmosphere.

With this configuration, the fluidic actuator 6 applies two different pressures within the internal side of the tip, namely a positive pressure (also referred to hereinafter as "dispensing pressure") that imposes the liquid inside the tip to flow out the tip **17** and makes the particles leaving the sensing tip, and a holding pressure as described above.

The dispensing pressure is chosen to avoid a too fast passage of particles through the sensing or restricted tip's membrane that would prevent the particles' detection. The holding pressure is chosen to counter act the hydrostatic pressure imposed by the particles' containing medium and creates a minimal flow entering the tip to avoid any particle leaving the sensing or restricted tip **17.** At the same time, the chosen holding pressure is such that it avoids the aspiration of particles from the outside of the sensing or restricted tip towards the inside.

In a preferred embodiment, the pumps **26, 27** used for the fluidic actuator are microfabricated piezo-actuated membrane pump. This kind of micropumps work by transferring a volume of fluid from one of their outlet to the other by moving a membrane. Their dead volume is small compared with the displaced volume and the membrane movement can be quick as it is piezo actuated. The combination of those element allows a precise control of the pressure in the sensing or restricted tip **17** as well as a fast switching of the fluidic actuator described above and required by the application. For example, it allows to switch from -2mbar to 2mbar in the sensing or restricted tip in less than 100ms.

When the system is operating, during the dispensing of the particles' comprising conductive medium located inside the pipette tip 17 of the invention towards an external reservoir filled with a conductive medium, also the inner hydrostatic pressure is consequently modified according to the change in total internal medium volume. As a consequence, both dispensing and holding pressures need to be adapted time after time based on the actual hydrostatic pressure.

Different solutions may be proposed to measure or estimate the hydrostatic pressure at each time point. For instance, in one embodiment, a pressure sensor 29 is placed in the proximity of the pipette tip membrane 3 in order to measure the inner hydrostatic pressure. In an alternative or additional embodiment, easier to fabricate, the internal electrode is not fully immersed into the particles' comprising conductive medium, and measuring its capacitance allow the determination of the internal medium height (and therefore, the internal hydrostatic pressure). In a further alternative or additional embodiment, by knowing beforehand, after a calibration of the restricted or sensing tip, the dispensed volume of the internal conductive medium overtime (e.g., dispensed microliters of particles' containing medium per second), the measurement of the time spent in the dispensing and holding state allows to estimate the tip's internal conductive medium volume. In an additional embodiment, a fluidic actuator 6 of any kind can be operated and regulated by an external controller, such as a pressure flow controller, a vacuum flow controller, a mechanical flow controller and the like, which can be embedded in a higher robotic system and possibly handled by a computer-like device.

## IMPEDANCE ANALYSER

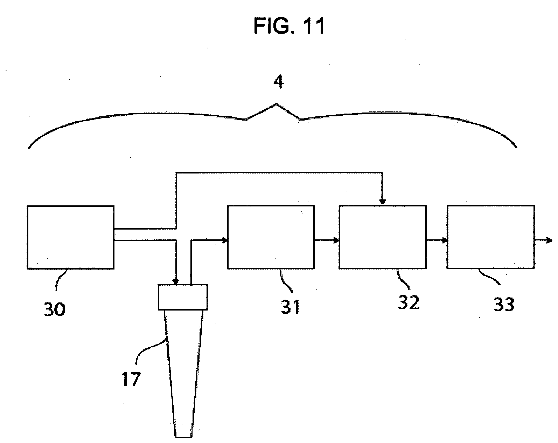

In order to produce an impedance signal, possibly interpretable by a controller 5, the impedance has to be read from the restricted or sensing tip of the invention (Fig. 11). For this purpose, an impedance analyser 4 is operably connected to the restricted or sensing tip through a tip connector. In a particular case and to allow the integration of the full system in a handheld device, this impedance analyser can be constituted of e.g. commercial integrated circuits following the well-known lock-in demodulator principle with the following block: a) an excitation source 30 at a single frequency or at multiple (at least two) frequencies for discrimination between e.g. two different particle types (e.g. cells and other particles) or different parameters of the same particle type such as for example cell viability; b) a preamplifier 31 to amplify the current received from the restricted or sensing tip 17; c) a demodulator 32 to extract the impedance signal from the modulated signal and d) a filter 33 to remove the harmonic created during the demodulation as well as any unwanted frequency from the impedance signal such as variation of medium conductivity.

One of the main features and advantages of the proposed system is actually the use of an impedance analyser 4 for recording an impedance signal, and store said information in an associated computer-like device as for example a log file. In cell dispensing procedures into multi-well plates for cell culture, this possibility has huge implications. In fact, saving in a file the impedance records together with the well index is an outstanding tool for process traceability. The traceability is a key feature of the system of the invention, which can be exploited to perform a quality control of the single-cell dispensing. Therefore, after a dispensing process, the impedance files of each well are retrieved and analysed by post-processing on a computer. If one single impedance peak is detected, the well is considered to have one single cell with high confidence.

## CONTROLLER

In at least some embodiments of the system of the invention, particularly those embodiments in which the system is intended for complete or semi-automatization, an optional, additional controller element 5 can operate the full system. In its simplest embodiment, a controller 5 is a computer-like device or an embedded micro-computer e.g. microcontroller operably connected to a fluidic actuator 6 and an impedance analyser 4 of the disclosed system, which is programmed with an algorithm in order to manage all the actions of the system.

Ideally, a controller 5 receives an impedance signal coming from the impedance analyser 4 and applies post-processing treatment on this signal that consists in a high pass filtering to remove the mean value of the signal. The controller 5 then performs a peak detection to reveal the event of particle's passage through the orifice 3 defined in the membrane 2 of the restricted or sensing tip. For example, in one embodiment the method used to perform this peak detection on the impedance signal is the comparison of the impedance signal with a given threshold representing the size of a particle. In another embodiment, the peak detection is performed by a correlation between the filtered impedance signal and signal representing the passage of a particle. In one embodiment, the controller 5 can furthermore receive instructions from the user in order to start different operational such as fluid aspiration or particle dispensing. Finally, the controller 5 drives the fluidic actuator according to a method suited for different operation such as cells or particles dispensing and this down to the resolution of one single cell.

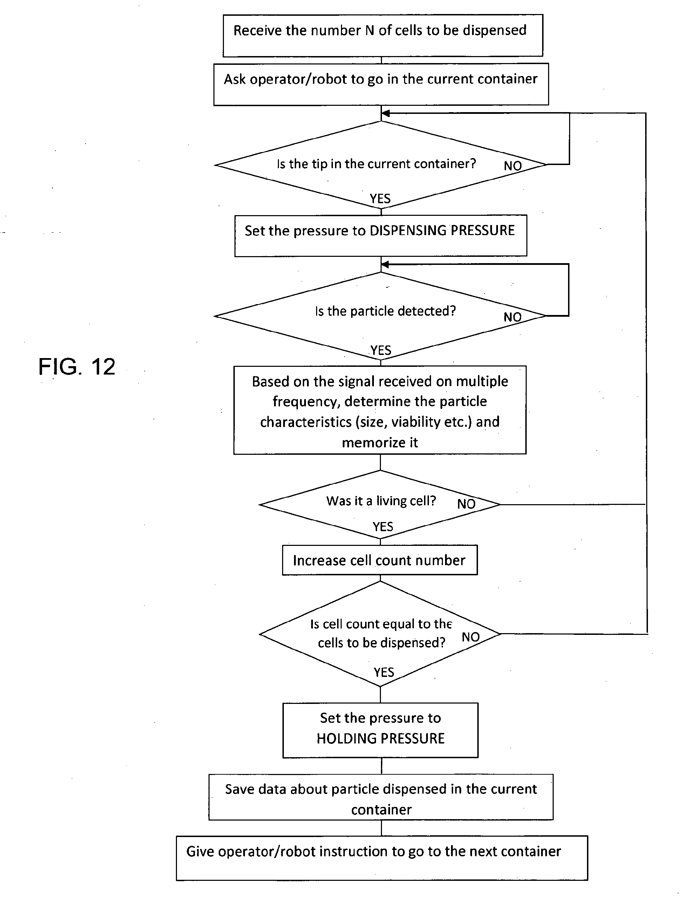

For an exemplary purpose only, an operation that can be performed by the system of the invention is the dispensing of N particles (with N ≥1) (Fig. 12), such as a cell, in an external reservoir comprising a conductive medium and fluidically connected to the pipette tip of the system.

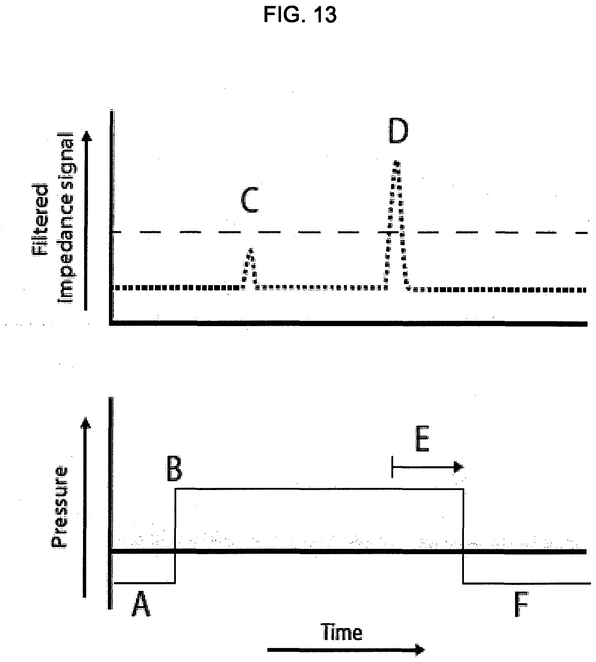

As depicted in Fig. 13, in a first step that is triggered by the user, particles in a conductive medium are loaded inside the restricted or sensing tip that is later operably connected to the fluidic actuator 6, preferably through a tip connector. For this step, the controller 5 activates the fluidic actuator 6 to generate a negative pressure for a given time to fill the sensing tip with a particles containing conductive medium. The controller 5 then places the system in an idle state (A) with the fluidic actuator 6 applying a holding pressure matching at least the hydrostatic pressure represented by the liquid within the restricted or sensing tip to avoid the exit of the medium with particles outside of the tip. Once the user has placed the restricted or sensing tip in the medium containing reservoir where the particle has to be dispensed, so that the pipette tip and the reservoir are fluidically and electrically connected, and the user has commanded the particles' dispensing (B), the controller 5 drives the fluidic actuator 6 so to generate a positive dispensing pressure to the restricted sensing tip. The conductive medium and the contained particles then start to flow out of the restricted or sensing tip through the orifice 3. Once the controller 5 detects the passage of a particle based on the signal received from the impedance analyser (D), the controller 5 waits a certain delay to make sure that the particle is not reaspirated and then operates so that a negative holding pressure is applied again by the fluidic controller (E). The system restarts the dispensing process (B) to reach the number N of particles to be dispensed and then waits again in the idle state (F) to receive further commands for particle dispensing.

As will be evident to a person skilled in the art, the inclusion of a controller 5 renders the entire system of the invention easily automated/automatable. In this context, additional elements such as robotic arms, displaceable platforms, optical sensors and the like can be included in the system; for instance, in cell dispensing procedures into multi-well plates for cell culture, a fluidic actuator 6 in the form of a pipettor, equipped with sensing or restricted tips according to the invention, can suitably be coupled with a robotic arm that moves according to a dispensing path along the wells of the plate, and based on the instructions provided by the controller 5. The robotic arm moves and displaces the pipettor 6, inserting and removing the tip from a first well, and repeating the operation cycle for any other well of the well-plate. Alternatively, the well plate can be moved along a pre-set path through a displaceable platform support, and the robotic arm simply inserts/removes the sensing tip into the wells by lifting and lowering the coupled pipettor 6.

## EXAMPLES

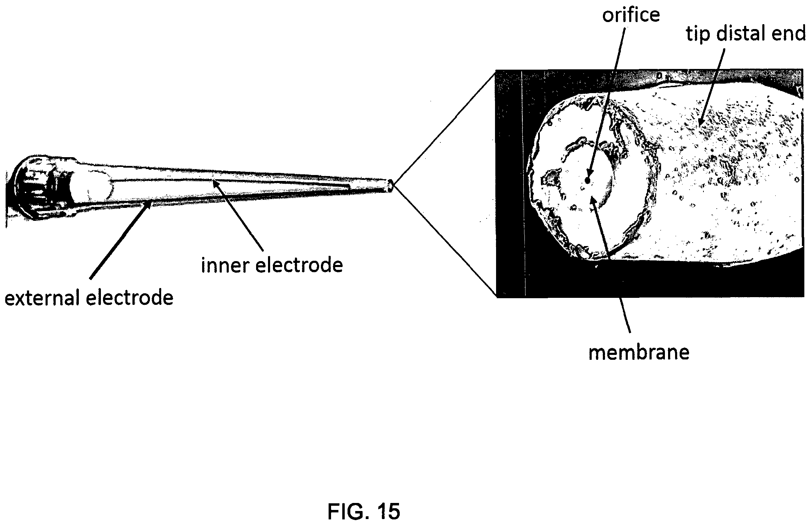

Figure 15 shows one embodiment of a sensing tip according to the invention. In this exemplary form, a 15$\mu$m Parylene C membrane is deposited by chemical vapour deposition (CVD) (C-30-S, Comelec, CH) on a commercial plastic pipette tip having a distal end (D) of 500 $\mu$m in diameter (200 $\mu$l colorless, RatioLab, GE). A 100nm thick gold outer electrode is subsequently deposited on the tip by evaporation (LAB 600H, Leybold Optics, CH). An orifice is then micromachined by excimer laser (LSV3, Optec, BE) on the previously deposited membrane. This is designed like a channel across a thin membrane restricting the tip opening. In order to optimize the sensor sensitivity and to avoid clogging, the orifice diameter (d) is of 30 $\mu$m, giving a D/d ratio of 16,6.

The depth of the orifice, which is equivalent to the membrane thickness, is of 15 $\mu$m. An inner electrode placed within the body of the pipette tip consists of a stainless steel wire. For sterility purposes and to block the internal electrode at a fixed height inside the tip, a high-density polyethylene filter (TipOne 200 $\mu$L, StarLab GE) is added to the tip from its top. The precise placement of the electrode being not critical, it is even possible to place the electrode manually, so that all the elements (the tip, the filter and the electrode) can be manually assembled under laminar flow.

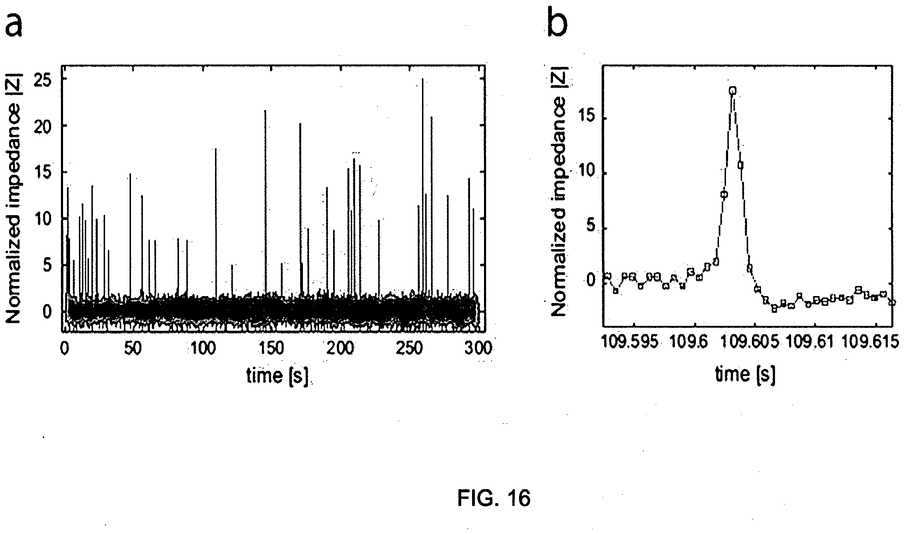

To test whether the so-obtained sensing tip is capable of detecting particles, a suspension of $10^4$ beads / mL as a surrogate of small cells was loaded in the tip (6-$\mu$m polystyrene beads). Once loaded, the sensing tip was placed in a tube containing fresh medium. The system was then set to flow some beads out for a period of 10 s, while recording of the impedance was performed. Figure 16 shows the impedance measurements graph acquired with the system of the invention implemented with a sensing tip.

## Claims

1. A pipette tip comprising:

> a) a proximal end possibly equipped with a sterility filter;
> b) an elongated body (1) adapted to retain a particle-containing conductive medium; and
> c) a distal end having a flow opening being closed at its extremity by a membrane (2) having a single orifice (3) thereon adapted to dispense a particle-containing conductive medium and particles comprised therein, said orifice having a diameter comprised between 1$\mu$m and 1000 $\mu$m

> **characterized in that** the membrane diameter/orifice diameter ratio is of at least 6.32, preferably at least 10.

2. The pipette tip of claim 1, **characterized in that** the proximal end surface area/distal end surface area ratio is at least 5.

3. The pipette tip of claim 1 or 2, **characterized in that** the membrane's diameter/membrane's thickness ratio is at least 5.

4. The pipette tip of any previous claim, **characterized in that** it has its proximal end adapted to be operably connectable with both a fluidic actuator (6) and an electrical impedance analyzer (4).

5. The pipette tip of any previous claim, **characterized in that** it comprises at least one electrode physically connected to a portion of the elongated body (1) and/or of the distal end and/or of the membrane (2) and/or of a sterility filter, said electrode being electrically activated once the pipette tip is operably connected to an electrical impedance analyzer (4).

6. A process for manufacturing a plastic pipette tip comprising a proximal end, an elongated body (1) adapted to retain a particle-containing conductive medium and a distal end having a flow opening being closed at its extremity by a membrane (2) having a single orifice (3) thereon having a diameter comprised between 1 $\mu$m and 1000 $\mu$m, wherein the membrane diameter/orifice diameter ratio is of at least 6.32, said process comprising the steps of:

   a) injecting a liquid plastic material into a mold defining the elongated body's (1) walls, form and thickness so to obtain a plastic pipette tip;
   b) providing a closed membrane (2);
   c) opening a single orifice (3) on the membrane (2);
   d) sealing the membrane (2) to the distal end of the plastic pipette tip; and
   e) optionally applying at least one electrode on the plastic pipette tip so that this latter can be operably connectable with an electrical impedance analyzer (4)

   wherein steps a) and steps b)-c) together are interchangeable.

7. A process for manufacturing a plastic pipette tip comprising a proximal end, an elongated body (1) adapted to retain a particle-containing conductive medium and a distal end having a flow opening being closed at its extremity by a membrane (2) having a single orifice (3) thereon having a diameter comprised between 1 $\mu$m and 1000 $\mu$m, wherein the membrane diameter/orifice diameter ratio is of at least 6.32, said process comprising the steps of:

   a) injecting a liquid plastic material into a mold defining the elongated body's (1) walls, form and thickness so to obtain a plastic pipette tip;
   b) temporary closing the distal end of the so-obtained plastic pipette tip;
   c) depositing a film of a plastic material on the elongated body's (1) walls and the distal end of the plastic pipette tip, thus defining a membrane (2) at the extremity of said distal end;
   d) removing the closure at the distal end of the plastic pipette tip;
   e) opening a single orifice (3) on the membrane (2); and
   f) optionally applying at least one electrode on the plastic pipette tip so that this latter can be operably connectable with an electrical impedance analyzer (4).

8. A system for use in dispensing and analysing particles through impedance-based means, **characterized in that** it comprises:

   a) a pipette tip of claims 1 to 5;
   b) an electrical impedance analyzer (4) adapted to be operably connectable to said pipette tip;
   c) a fluidic actuator (6) adapted to be operably connectable to the proximal end of the pipette tip; and
   d) optionally a controller (5) adapted to be operably connectable to, and operate, both the fluidic actuator (6) and the electrical impedance analyzer (4).

9. The system of claim 8, further comprising a computer device operably connected to the electrical impedance analyzer (4) for analysing and/or store impedance data.

10. The system of claims 8 or 9, **characterized in that** the fluidic actuator (6) comprises two pressure sources and one regulator for modifying the pressure inside the pipette tip.

11. The system of claims 8 to 10, **characterized in that** the fluidic actuator (6) comprises at least two air pumps operably connected to the pipette tip through a 3-ways valve adapted so that at least one pump generates a positive pressure and at least one pump generates a negative pressure inside the pipette tip.

**12.** The system of claims 8 to 11, **characterized in that** it further comprises a hydrostatic pressure sensor placed within the pipette tip and in proximity of the orifice (3).

**13.** The system of claims 8 to 12, **characterized in that** the controller (5) regulates the fluidic actuator (6) activity in response to the measurements performed by the electrical impedance analyzer (4).

**Patentansprüche**

**1.** 1. Pipettenspitze, umfassend:

a) ein proximales Ende, das möglicherweise mit einem Sterilfilter ausgestattet ist;
b) einen länglichen Körper (1), der zum Aufnehmen eines teilchenhaltigen leitenden Mediums ausgelegt ist; und
c) ein distales Ende mit einer Durchflussöffnung, das an seinem äußersten Ende durch eine Membran (2) mit einem einzigen Loch (3) darin verschlossen ist, das zum Ausgeben eines teilchenhaltigen leitenden Mediums und darin enthaltener Teilchen ausgelegt ist, wobei das Loch einen Durchmesser zwischen 1 $\mu$m und 1000 $\mu$m aufweist,

**dadurch gekennzeichnet, dass** das Verhältnis von Membrandurchmesser zu Lochdurchmesser mindestens 6,32, vorzugsweise mindestes 10 beträgt.

**2.** Pipettenspitze nach Anspruch 1, **dadurch gekennzeichnet, dass** das Verhältnis des Oberflächenbereichs des proximalen Endes zum Oberflächenbereich des distalen Endes mindestens 5 beträgt.

**3.** Pipettenspitze nach Anspruch 1 oder 2, **dadurch gekennzeichnet, dass** das Verhältnis von Membrandurchmesser zu Membrandicke mindestens 5 beträgt.

**4.** Pipettenspitze nach einem der vorhergehenden Ansprüche, **dadurch gekennzeichnet, dass** sie ihr proximales Ende so aufweist, dass es sowohl mit einem Fluidaktor (6) als auch einem elektrischen Impedanzanalysator (4) funktionell verbunden werden kann.

**5.** Pipettenspitze nach einem der vorhergehenden Ansprüche, **dadurch gekennzeichnet, dass** sie mindestens eine Elektrode aufweist, die mit einem Abschnitt des länglichen Körpers (1) und/oder des distalen Ende und/oder der Membran (2) und/oder eines Sterilfilters physisch verbunden ist, wobei die Elektrode elektrisch aktiviert wird, sobald die Pipettenspitze mit einem elektrischen Impedanzanalysator (4) verbunden wird.

**6.** Prozess zur Herstellung einer Pipettenspitze aus Kunststoff mit einem proximalen Ende, einem länglichen Körper (1), der zur Aufnahme eines teilchenhaltigen leitenden Mediums ausgelegt ist, und einem distalen Ende mit einer Durchflussöffnung, das an seinem äußersten Ende durch eine Membran (2) mit einem einzigen Loch (3) darin mit einem Durchmesser zwischen 1 $\mu$m und 1000 $\mu$m verschlossen ist, wobei das Verhältnis von Membrandurchmesser zu Lochdurchmesser mindestens 6,32 beträgt,
wobei der Prozess die folgenden Schritte umfasst:

a) Injizieren eines flüssigen Kunststoffmaterials in ein Formwerkzeug, das die Wände, Form und Dicke des länglichen Körpers (1) definiert, um auf diese Weise eine Pipettenspitze aus Kunststoff zu erhalten;
b) Bereitstellen einer geschlossenen Membran (2);
c) Öffnen eines einzigen Lochs (3) in der Membran (2) ;
d) Versiegeln der Membran (2) am distalen Ende der Pipettenspitze aus Kunststoff; und
e) optionales Anbringen mindestens einer Elektrode auf der Pipettenspitze aus Kunststoff, so dass diese später mit einem elektrischen Impedanzanalysator (4) funktionell verbunden werden kann,

wobei Schritt a) und Schritt b) bis c) untereinander austauschbar sind.

**7.** Prozess zur Herstellung einer Pipettenspitze aus Kunststoff mit einem proximalen Ende, einem länglichen Körper (1), der zur Aufnahme eines teilchenhaltigen leitenden Mediums ausgelegt ist, und einem distalen Ende mit einer Durchflussöffnung, das an seinem äußersten Ende durch eine Membran (2) mit einem einzigen Loch (3) darin mit einem Durchmesser zwischen 1 $\mu$m und 1000 $\mu$m verschlossen ist, wobei das Verhältnis von Membrandurchmesser zu Lochdurchmesser mindestens 6,32 beträgt,

wobei der Prozess die folgenden Schritte umfasst:

a) Injizieren eines flüssigen Kunststoffmaterials in ein Formwerkzeug, das die Wände, Form und Dicke des länglichen Körpers (1) definiert, um auf diese Weise eine Pipettenspitze aus Kunststoff zu erhalten;

b) temporäres Schließen des distalen Endes der auf diese Weise erhaltenen Pipettenspitze aus Kunststoff;

c) Aufbringen eines Films aus Kunststoffmaterial auf die Wände des länglichen Körpers (1) und das distale Ende der Pipettenspitze aus Kunststoff, um dadurch eine Membran (2) am äußersten Ende des distalen Endes zu definieren;

d) Entfernen des Verschlusses am distalen Ende der Pipettenspitze aus Kunststoff;

e) Öffnen eines einzigen Lochs (3) in der Membran (2); und

f) optionales Anbringen mindestens einer Elektrode auf der Pipettenspitze aus Kunststoff, so dass diese später mit einem elektrischen Impedanzanalysator (4) funktionell verbunden werden kann,

8. System zur Verwendung beim Ausgeben und Analysieren von Teilchen durch impedanzbasierte Mittel, **dadurch gekennzeichnet, dass** es umfasst:

a) eine Pipettenspitze nach einem der Ansprüche 1 bis 5;

b) einen elektrischen Impedanzanalysator (4), der so ausgelegt ist, dass er mit der Pipettenspitze funktionell verbunden werden kann;

c) einen Fluidaktor (6), der so ausgelegt ist, dass er mit dem proximalen Ende der Pipettenspitze verbunden werden kann; und

d) optional eine Steuerung (5), die so ausgelegt ist, dass sie sowohl mit dem Fluidaktor (6) als auch dem elektrischen Impedanzanalysator (4) funktionell verbunden werden kann und diese steuert.

9. System nach Anspruch 8, ferner umfassend eine Computervorrichtung, die mit dem elektrischen Impedanzanalysator zum Analysieren und/oder Speichern von Impedanzdaten (4) funktionell verbunden ist.

10. System nach Anspruch 8 oder 9, **dadurch gekennzeichnet, dass** der Fluidaktor (6) zwei Druckquellen und einen Regler zum Modifizieren des Drucks innerhalb der Pipettenspitze umfasst.

11. System nach einem der Ansprüche 8 bis 10, **dadurch gekennzeichnet, dass** der Fluidaktor (6) mindestens zwei Luftpumpen umfasst, die mit der Pipettenspitze durch ein 3-Wege-Ventil funktionell verbunden sind, das so ausgelegt ist, dass mindestens eine Pumpe einen Überdruck und mindestens eine Pumpe einen Unterdruck innerhalb der Pipettenspitze erzeugt.

12. System nach einem der Ansprüche 8 bis 11, **dadurch gekennzeichnet, dass** es ferner einen hydrostatischen Drucksensor umfasst, der innerhalb der Pipettenspitze und in der Nähe des Lochs (3) angeordnet ist.

13. System nach einem der Ansprüche 8 bis 12, **dadurch gekennzeichnet, dass** die Steuerung (5) die Aktivität des Fluidaktors (6) in Reaktion auf Messungen regelt, die durch den elektrischen Impedanzanalysator (4) durchgeführt werden.

## Revendications

1. Pointe de pipette comprenant :

a) une extrémité proximale éventuellement équipée d'un filtre de stérilité ;

b) un corps allongé (1) adapté pour retenir un milieu conducteur contenant des particules ; et

c) une extrémité distale comportant une ouverture d'écoulement fermée à son extrémité par une membrane (2) comportant un seul orifice (3) adapté pour distribuer un milieu conducteur contenant des particules et les particules qui y sont comprises, ledit orifice ayant un diamètre compris entre 1 $\mu$m et 1000 $\mu$m

**caractérisée en ce que** le rapport diamètre de la membrane/diamètre de l'orifice est d'au moins 6,32, de préférence d'au moins 10.

2. Pointe de pipette selon la revendication 1, **caractérisée en ce que** le rapport surface d'extrémité proximale/surface d'extrémité distale est d'au moins 5.

3. Pointe de pipette selon la revendication 1 ou 2, **caractérisée en ce que** le rapport diamètre de la membrane/épaisseur de la membrane est d'au moins 5.

4. Pointe de pipette selon n'importe quelle revendication précédente, **caractérisée en ce qu'**elle a son extrémité proximale adaptée pour être connectée de manière opérationnelle à la fois à un actionneur fluidique (6) et à un analyseur d'impédance électrique (4).

5. Pointe de pipette selon n'importe quelle revendication précédente, **caractérisée en ce qu'**elle comprend au moins une électrode physiquement connectée à une partie du corps allongé (1) et/ou de l'extrémité distale et/ou de la membrane (2) et/ou d'un filtre de stérilité, ladite électrode étant électriquement activée une fois que la pointe de pipette est connectée de manière opérationnelle à un analyseur d'impédance électrique (4).

6. Procédé de fabrication d'une pointe de pipette en plastique comprenant une extrémité proximale, un corps allongé (1) adapté pour retenir un milieu conducteur contenant des particules et une extrémité distale ayant une ouverture d'écoulement fermée à son extrémité par une membrane (2) ayant un seul orifice (3) sur celle-ci, ayant un diamètre compris entre 1 $\mu$m et 1000 $\mu$m, le rapport diamètre de la membrane/diamètre de l'orifice étant d'au moins 6,32, ledit procédé comprenant les étapes de :

   a) injection d'une matière plastique liquide dans un moule définissant les parois, la forme et l'épaisseur du corps allongé (1) de manière à obtenir une pointe de pipette en plastique ;
   b) fourniture d'une membrane fermée (2) ;
   c) ouverture d'un seul orifice (3) sur la membrane (2) ;
   d) scellement de la membrane (2) à l'extrémité distale de la pointe de pipette en plastique ; et
   e) application éventuellement d'au moins une électrode sur la pointe de pipette en plastique de sorte que cette dernière peut être connectée de manière opérationnelle à un analyseur d'impédance électrique (4) les étapes a) et b)-c) ensemble étant interchangeables.

7. Procédé de fabrication d'une pointe de pipette en plastique comprenant une extrémité proximale, un corps allongé (1) adapté pour retenir un milieu conducteur contenant des particules et une extrémité distale ayant une ouverture d'écoulement fermée à son extrémité par une membrane (2) ayant un seul orifice (3) sur celle-ci, ayant un diamètre compris entre 1 $\mu$m et 1000 $\mu$m, le rapport diamètre de la membrane/diamètre de l'orifice étant d'au moins 6,32, ledit procédé comprenant les étapes de :

   a) injection d'une matière plastique liquide dans un moule définissant les parois, la forme et l'épaisseur du corps allongé (1) de manière à obtenir une pointe de pipette en plastique ;
   b) fermeture temporairement de l'extrémité distale de la pointe de pipette en plastique ainsi obtenu ;
   c) dépôt d'un film de matière plastique sur les parois du corps allongé (1) et l'extrémité distale de la pointe de pipette en plastique, définissant ainsi une membrane (2) à l'extrémité de ladite extrémité distale ;
   d) retrait de la fermeture à l'extrémité distale de la pointe de pipette en plastique ;
   e) ouverture d'un seul orifice (3) sur la membrane (2) ; et
   f) application éventuellement d'au moins une électrode sur la pointe de pipette en plastique de sorte que cette dernière peut être connectée de manière opérationnelle à un analyseur d'impédance électrique (4).

8. Système destiné à être utilisé pour distribuer et analyser des particules par des moyens basés sur l'impédance, **caractérisé en ce qu'**il comprend :

   a) une pointe de pipette selon les revendications 1 à 5 ;
   b) un analyseur d'impédance électrique (4) adapté pour être connecté de manière opérationnelle à ladite pointe de pipette ;
   c) un actionneur fluidique (6) adapté pour être connecté de manière opérationnelle à l'extrémité proximale de la pointe de pipette ; et
   d) éventuellement un dispositif de commande (5) adapté pour être connecté de manière opérationnelle à, et faire fonctionner, à la fois l'actionneur fluidique (6) et l'analyseur d'impédance électrique (4).

9. Système selon la revendication 8, comprenant en outre un dispositif informatique connecté de manière opérationnelle à l'analyseur d'impédance électrique (4) pour analyser et/ou stocker des données d'impédance.

10. Système selon les revendications 8 ou 9, **caractérisé en ce que** l'actionneur fluidique (6) comprend deux sources de pression et un régulateur pour modifier la pression à l'intérieur de la pointe de pipette.

11. Système selon les revendications 8 à 10, **caractérisé en ce que** l'actionneur fluidique (6) comprend au moins deux pompes à air connectées de manière opérationnelle à la pointe de pipette par l'intermédiaire d'une vanne à 3 voies adaptée de sorte qu'au moins une pompe génère une pression positive et au moins une pompe génère une pression négative à l'intérieur de la pointe de pipette.

12. Système selon les revendications 8 à 11, **caractérisé en ce qu'**il comprend en outre un capteur de pression hydrostatique placé à l'intérieur de la pointe de pipette et à proximité de l'orifice (3).

13. Système selon les revendications 8 à 12, **caractérisé en ce que** le dispositif de commande (5) régule l'activité de l'actionneur fluidique (6) en réponse aux mesures effectuées par l'analyseur d'impédance électrique (4).

FIG. 1

## FIG. 2

# FIG. 3

# FIG. 4

## FIG. 5

## FIG. 6

# FIG. 7

# FIG. 8

# FIG. 9

# FIG. 10

FIG. 11

**FIG. 12**

Receive the number N of cells to be dispensed

Ask operator/robot to go in the current container

Is the tip in the current container?    NO

YES

Set the pressure to DISPENSING PRESSURE

Is the particle detected?    NO

YES

Based on the signal received on multiple frequency, determine the particle characteristics (size, viability etc.) and memorize it

Was it a living cell?    NO

YES

Increase cell count number

Is cell count equal to the cells to be dispensed?    NO

YES

Set the pressure to HOLDING PRESSURE

Save data about particle dispensed in the current container

Give operator/robot instruction to go to the next container

27

FIG. 13

## FIG. 14

**FIG. 15**

a

b

FIG. 16

EP 3 283 221 B1

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2656508 A
- US 3714565 A
- WO 2005121780 A2, Gascoyne
- WO 2010093998 A2
- EP 0494735 A